# EUROPEAN PATENT SPECIFICATION

(11) **EP 1 374 903 B1**
(45) Date of publication and mention of the grant of the patent: **28.03.2007**
(21) Application number: 02425423.7
(22) Date of filing: 26.06.2002
(51) Int. Cl.: A61K 47/32, A61K 31/164, A61K 31/78, A61K 31/13, A61K 31/19, A61K 31/4188, A61K 31/085, A61K 31/047, A61P 31/10

(54) **Pharmaceutical composition for the prevention of the development and progression of mycotic skin surface diseases**
Pharmazeutische Zusammensetzung zur Verhinderung der Entwicklung und Progression von mykotischen Hautoberflächenerkrankungen
Composition pharmaceutique pour prévenir le développement et la progression de mycoses cutaneés supérieures

(43) Date of publication of application: 02.01.2004
(73) Proprietor: Innovet Italia S.r.l., 20144 Milano (IT)
(72) Inventor: Della Valle, Francesco, 35122 Padova (IT); Della Valle, Maria Federica, 35141 Padova (IT); Comelli, Maria Cristina, 35141 Padova (IT)
(74) Representative: Long, Giorgio

(56) References cited:
- WO-A-01/04083
- WO-A-01/10402
- WO-A-96/18600
- MAYSER P; IMKAMPE A; WINKELER M; PAPAVASSILIS C: "Growth requirements and nitrogen metabolism of Malassezia furfur" ARCH DERMATOL RES, vol. 290, no. 5, May 1998 (1998-05), pages 277-282, XP002224820

## Description

This invention refers to pharmaceutical compositions applicable for the prevention of the development and progression of mycotic skin surface diseases in human and animal, in particular of those associated with the proliferation and conversion of commensal lipophile fungi, such as for instance the genus *Malassezia*/*Pityrosporum.*

Pityriasis versicolor (PV) is a mycotic surface disease generally limited to to corneal layer, which clinically manifests itself by the appearance of maculae or slightly desquamating spots, depigmented or hyperpigmented, erythematous and frequently associated with itching. The most commonly affected points are the upper parts of the trunk, the shoulders, the upper surface of the arms, the neck and occasionally the abdomen (Assaf RR, Weil ML, 1996, Dermatol Clin., 14: 57-67). PV prevalently affects the male population comprised between 20 and 40 years; it has a higher incidence in hot climates, in patients affected by seborrhea, hyperhidrosis, and in patients with immunity ailments. Generally without symptoms, the infection is frequently recognized during the summer months, by the affected skin surfaces' inability to brown in the sun. PV is determined by the pathogenous colonization of the corneal layer on the part of the ifal form of a dimorphic lipophilic yeast, a saprophite belonging to the genus *Malassezia* sp.. On a molecular level, the genus *Malassezia* comprises various species, all of which are normal constituents of the microbic flora resident on human and/or animal skin. The various species of *Malassezia* are dimorphic, meaning characterized by a yeast phase in quiescent periods and by the conversion to a myceliar form with development of a hyphal in the active infection phase. The *Malassezia* species are further involved in the development of other surface dermatosis, such as seborrhoic dermatitis (chronic desquamating dermatitis of the face and upper trunk), folliculitis, and in the exacerbation of eczematous reactions of atopic dermatitis (Watanabe S. et al., 2001, J. Invest. Dermatol., 166:769-773). It is also held that *Malassezia* is an exogenous triggering factor for common psoriatic lesions (Schmidt A., 1997, Cutis, 59:21-24). A particular species of *Malassezia,* the typically zoophilic *M. pachydermatis,* is responsible in cats and dogs for two clinically well defined pictures: a) an itching dermatitis - a dermatitis from *Malassezia -* which manifests itself in dogs of every age and sex, with a certain predisposition toward races such as the WHWT, the Basset Hound, the Maltese, the small Poodle and the Shar Pei. It is characterized by the appearance of erithematous papules/spots which are initially localicalized at an abdominal level and subsequently in the groin, armpits and neck region, and by keratoseborrhoic alterations including hyperpigmentation, desquamation, alopecia and an alternatingly dry/crusty or oily appearance of the skin and fur; b) a usually bilateral external otitis, of an erithematous/ ceruminous type, which is practically constantly accompanied by edema and pain. Normally present as a commensal on the skin, *M. pachydermatis* transforms itself into opportunistic pathogenous yeast due to numerous factors, both genetic (for instance in the presence of cutaneous plicae, primary idiopathic seborrhoea, immuno-deficiencies) and acquired (for instance allergic diseases, pyodermitic infections, ectoparassitoses, cheratinization problems, dysendocrinias, prolonged treatments with glucocorticoids and/or antibiotics). The commonly known therapy is based on the use of topic preparations, such as synthetic detergents (for instance, lotions and shampoos), containing antimycotics such as for instance climbazol, ketoconazol, poctone-olamine, zinc pyrithione, selenium sulphide or other sulfur-containing substances. Gels or creams containing azolinic antimycotics or cyclopirox-olamine are also used. These topic antifungal preparations are often combined with a cortisonic drug to control the inflammation and alleviate the pain and itching. However, the use of molecules esxclusively aimed at a fungistatic/fungicidal action, even if effective in containing the acute infective phase, by directly acting on the pathogenic microorganism, they do not result fully satisfactory. These molecules exhibit in fact a high intrinsic toxicity. At this time, for instance, the preventive topic therapy with shampoos or lotions based on 2.5% selenium sulphide cannot be prescribed to patients with a reddened and unhealthy skin, due to their toxicity. These types of treatment are moreover always associated with a high incidence of recurrence of skin surface mycoses, independently of the regimen of treatment adopted in the active phase. For instance, the relapse rate of acute mycosis is estimated at 20% of the population affected by PV and treated with ketoconazol within 6 months from the date of suspending the therapy, and at 50% after one year.

The technical problem solved by this invention is therefore to make available a new therapeutic approach aimed at the prevention and treatment of skin surface mycosis supported by strains of lipphile yeasts, so as to solve the problems of chronicity and the high rate of relapse observed in the therapies already known.

This problem is resolved by a composition such as that described in the attached claims.

The composition object of this invention comprises a mixture of an agonist of the receptor of type CB2 of the cannabinoids and of a free fatty acid sequestering resin. This composition was found to be particularly useful in curing skin surface mycoses, thanks to the synergic action of the against of the receptor of type CB2 of the cannabinoids which is particularly active as a normalizer of the hyper-reactivity of dermo-epidermic tissue, and of the resin with a lipoabsorbant action, which is capable of sequestering the nutrient substrate from the yeast, thus rebalancing the microbial flora.

Without wishing to be bound to any particular theory, it was found that from a histological viewpoint the development of surface mycoses, such as for instance PV, results from an altered responsiveness of the guest towards the resident flora, with the consequent development of an altered epidermic reactivity towards the lipphile yeast-like micetes. The pathogenetic mechanism underlying the PV development resides therefore in a local inflammatory response primed by toxins or mediators induced and/or produced by *Malassezia*/*Pityrosporum,* and by the fungus lipasic activity. It has moreover been demonstrated, especially in the dog, that *M. pachydermatis* induces a peculiar distribution of the skin mastocytes, which migrate from a deep-seated to a surface derma and align themselves at the base of the epidermis (the so-called Subepidermal Linear Alignement of Mast Cells, or SLAM). This means that *Malassezia*/*Pityrosporum* induces a state of mastocyte hyper-reactivity, with an increase of not merely the degranulation of the pro-inflammatory and cytodamaging mediators but also of the intratissuar migratory capacity. The *Malassezia*/*Pityrosporum* stimulate the epidermal keratinocytes to the production of sustained levels of pro-inflammatory cytokines. This results in an anomalous stimulus inducing a persistent dermo-epidermal hyper-activity, which contributes to the progressive loss of the barrier property and to an altered local lipidic metabolism, with a consequent alteration of the fungus' substrate of growth. All the above-mentioned conditions determine a substrate favourable to the growth and myceliar differentiation of the pathogen. In particular, the hyperactivity of the sebaceous glands, from which the secretion of the fatty acids needed for the development of *Malassezia* depends, plays a prime role. For this reason, in skin areas with a high sebacic production, such as the skin of the trunk and scalp, the hyphal production and differentiation are strongly facilitated, with the onset of an active infection and the development of epidermic lesions. The dermo-epidermic iper reactivity may further justify the high incidence of recurrence of superficial mycosis, independently of the treating regimen adopted in the active phase.

Without therefore directly acting on the pathogenous fungus, but interacting only with the pathogenetic mechanisms of the mycotic lesion, it has been surprisingly discovered by the inventors of this invention an innovative and effective form of intervention for the prevention and treatment of mycotic infections.

It was in fact found that the functional activation of the peripheric receptor of type CB2 of the cannabinoids expressed on the membrane of the mastocytes determines an inhibitory modulation of the degranulatory tone, with a progressive reduction of the mediators released. The functional agonists of the mastocyte receptor of type CB2 allow, by specifically acting on the hyper-active mastocyte, to rebalance the dermo-epidermic cytokinic balance altered by the *Malassezia*-dependent overstimulation, thus favoring the correct keratocytic metabolism and determine a significant reduction of the hyper-reactive and inflammatory signs and symptoms. The physiological recovery of the mastocytic degranulatory tone thus allows controlling the itching symptomatology and to oppose the onset and amplification of inflammatory skin conditions.

Based on this invention the compounds chosen as agonists of the cannabinoid receptor of type CB2 present on the mastocyte are covalent derivatives of alcanolamides of mono- and dicarboxylic acids with amminoalcohols of the general formula (I):

R₁-CO-NR₂R₃ (I)

These formula (I) compounds are described in the patent application WO 01/04083, whose relative description has been incorporated here for reference.

The formula (I) compounds preferably selected are those in which:
a) R₁ may be:
   - a linear or branched alkylic, an alkylenic or arylic radical.
   - a group of the formula:

      -R₄-CO-NR₅R₆
   wherein R₄ is a linear or branched alkylenic radical or an arylic biradical; R₅ and R₆ have the significance reported in the following for R₂ and R₃, respectively.
b) R₂ is chosen among hydrogen or an alkylic, alkenylic or arylic radical.
b) R₃ is a group of the formula:

   -Y-OH
wherein Y a linear or branched alkylenic radical.

More preferably, derivatives of alcanolamides of dicarboxylic acids with amino-alcohols of the general formula (II)are chosen:

HO-(CH₂)ₙ-NH-CO-(CH₂)ₘ-CO-NH-(CH₂)ₙ-OH (II)

Wherein n has a value comprised between 2 and 10 and m has a value comprised between 2 and 12; n is preferably a number comprised between 2 and 4 and m a number comprised between 5 and 9.

Even more preferably, the active principle selected to control the mastocyte-mediated skin hyper-activity is a compound of the formula (II), where n = 2 and m = 7, or the compound N-N¹-bis(2-hydroxyethyl)nonandiamide, whose Common International Denomination is Adelmidrol. Adelmidrol is a compound obtained in accordance with the process described in the patent application WO 01/10402, incorporated here for reference.

The selected resin is an acrylic copolymer charged with phytosphingosine. This resin is an ion exchange resin with a high affinity for free fatty acids, which are promptly absorbed on the surface of the copolymer. This deprives the *Malassezia,* a lipid-dependent yeast, of its optimal substrate for growth and differentiation.

Even more preferably the acrylic copolymer selected is POLYTRAP^{®} Q5-6603, commercialised by the firm Dow Corning.

In the composition according to this invention the agonist of the receptor of type CB2 of the cannabinoids and the fatty acid sequestering resin are present in a weight ratio variable from 3:1 to 1:6 respectivly, preferably from 1:1 to 1:2.

For the purpose of being able to optimise the antimycotic activity, the composition of this invention may additionally comprise one or more active substances chosen among:
- a substance capable of sustaining and enhancing the keratinocytic metabolism;
- a substance with a restructuring and bioadhesive action;
- a substance with an antimycotic action.

The active principle preferably selected for sustaining and enhancing the keratocytic metabolism is biotine. It facilitates the re-establishment of the balance between cellular renewal and esfoliation, a physiological mechanism of primary importance for eliminating surface microbial excesses. The biotine if present in the composition, will preferably have a weight ratio comprised between 1:50 and 1:250 with respect to the agonist of the receptor of type CB2 of cannabinoids.

The active principles with bioadhesive and restructuring activity are preferably selected among 2-dodecenedioic or traumatic acid and/or jaluronic acid. They maintain and return the keratinocytes in the damaged area to an adhesive condition, by blocking the access routes for the onset of fungine infection. The jaluronic acid and/or traumatic acid, if present in the composition, will preferably have a weight ratio comprised between 1:1.5 and 1:4 with respect to the agonist of the receptor of the CB2 type of the cannabinoids.

The antimycotics are preferably selected between echinacea purpurea, usnic acid, undecilenic acid, bronopol and/or triclosan. These substances are contained in the composition according to this invention at a much lower dose than normally utilized in a classic antimycotic used for resolving an acute infection. At these low concentrations, which are not such as to be capable of effecting a specifically pharmaceutic and therapeutically effective action to eliminate the pathogenous fungus, the substances with an antimycotic action perform a synergically controlling action on the bacterial flora.

The echinacea purpurea if present in the composition, will preferably have a weight ratio variable between 7:1 and 4:1 with respect to the against of the receptor of the CB2 type of the cannabinoids.

The usnic acid, if present in the composition, will preferably have a weight ratio variable between 1:5 and 1:7 with respect to the against of the receptor of the CB2 type of the cannabinoids.

The undecilenic acid if present in the composition, will preferably have a weight ratio variable between 1:8 and 1:12 with respect to the against of the receptor of the CB2 type of the cannabinoids.

The bronopol if present in the composition, will preferably have a weight ratio variable between 1:30 and 1:50 with respect to the against of the receptor of the CB2 type of the cannabinoids.

The triclosan if present in the composition, will preferably have a weight ratio variable between 1:5 and 1:8 with respect to the against of the receptor of the CB2 type of the cannabinoids.

The composition of this invention may also comprise some pharmaceutically acceptable excipients, chosen depending on the type of formulation and on the desired administration route.

The formulations will in particular be administered by a topic route, including a transdermic route, and comprise formulations in the form of creams, ointments, lotions, shampoos, sprays, gels or patches.

The pharmaceutical formulations are prepared in agreement with the normal methods used in pharmaceutical technology, which will therefore not be described in detail. The various pharmaceutical forms mentioned may also be formulated with excipients and/or by technological processes suitable for obtaining drugs having a modified release and preferably a prolonged action.

The therapeutically effective dosages of the compositions object of this invention may be different depending on the pharmaceutical form chosen and may be decided by the physician depending on the gravity of the pathology and on the physical conditions of the patient. Moreover, depending on the gravity of the pathology, the administration regimen may provides for one or more daily doses, for a variable time and can also be repeated in various cycles, as shall be determined by the treating physician based on his experiences.

The invention is further described with the aid of the following examples of a pharmaceutical composition which are not limiting it, as these purposes may also be served by other formulations obtained based on other ratios between the active principles and while using other excipients.

### EXAMPLES OF COMPOSITIONS

In all the examples to follow, the designation OE means "oxyethylated".

### Example 1 - Gel for application on skin and mucous membranes

100 g of gel contain:

| | | |
|---|---|---|
| • Acrylic copolymer | g | 2,50 |
| • Adelmidrol | g | 1.00 |
| • Echinacea purpurea extract | g | 15.00 |
| • Deo-usnate | g | 0.60 |
| • Jaluronic acid | g | 0.30 |
| • Trans-traumatic acid | g | 0.05 |
| • Phytosphingosine | g | 0.01 |
| • Triclosan | g | 0.30 |
| • Undecilenic acid | g | 0.20 |
| • Biotin | g | 0.01 |
| • Sodium alginate | g | 2.50 |
| • Carbomer | g | 0.60 |
| • Sodium hydroxide | g | 0.50 |
| • Water | g | 76,43 |

### Example 2 - Cream gel for application on skin

100 g of cream gel contain:

| | | |
|---|---|---|
| • Acrylic copolymer | g | 2,00 |
| • Adelmidrol | g | 2.00 |
| • Echinacea purpurea extract | g | 10.00 |
| • Deo-usnate | g | 0.60 |
| • Jaluronic acid | g | 0.20 |
| • Trans-traumatic acid | g | 0.01 |
| • Phytosphingosine | g | 0.01 |
| • Triclosan | g | 0.30 |
| • Undecilenic acid | g | 0.20 |
| • Biotin | g | 0.01 |
| •Hydrognated castor oil 40 (OE) | g | 30.00 |
| • Carbomer | g | 0.60 |
| • Sodium hydroxide | g | 0.50 |
| • Water | g | 80.52 |

### Example 3 - Emulsion for application on scalp

100 g of emulsion contain:

| | | |
|---|---|---|
| • Acrylic copolymer | g | 2,00 |
| • Cyclometicone | g | 5.00 |
| • Adelmidrol | g | 2.00 |
| • Echinacea purpurea extract | g | 10.00 |
| • Deo-usnate | g | 0.60 |
| • Jaluronic acid | g | 0.20 |
| • Trans-traumatic acid | g | 0.01 |
| • Phytosphingosine | g | 0.01 |
| • Triclosan | g | 0.30 |
| • Undecilenic acid | g | 0.20 |
| • Biotin | g | 0.01 |
| • Hydrogenated castor oil 40 (OE) | g | 30.00 |
| • Carbomer | g | 0.60 |
| • Sodium hydroxide | g | 0.50 |
| • Water | g | 79.60 |

### Example 4 - Emulsion for application in the ear duct of dogs

100 g of emulsion contain:

| | | |
|---|---|---|
| • Acrylic copolymer | g | 3.00 |
| • Cyclometicone | g | 7.00 |
| • Adelmidrol | g | 2.00 |
| • Echinacea purpurea extract | g | 10.00 |
| **•** Deo-usnate | g | 0.60 |
| • Jaluronic acid | g | 0.20 |
| • Trans-traumatic acid | g | 0.01 |
| • Phytosphingosine | g | 0.01 |
| • Triclosan | g | 0.30 |
| • Undecilenic acid | g | 0.20 |
| • Hydrogenated castor oil with/Peg 40 | g | 20.00 |
| • Polyethyleneglycol | g | 30.00 |
| • Water | g | 26,68 |

### Example 5 - Transdermic patches for application on skin

Every patch contains:

| | | |
|---|---|---|
| • Acrylic copolymer | g | 0.0040 |
| • Adelmidrol | g | 0.0030 |
| • Echinacea purpurea extract | g | 0.0030 |
| • Usnic acid | g | 0.0010 |
| • Trans-traumatic acid | g | 0.0002 |
| • Phytosphingosine | g | 0.0005 |
| • Triclosan | g | 0.0030 |
| • Undecilenic acid | g | 0.0050 |
| • Adhesive excipients | g | 0.0600 |

### Example 6 - Cosmetic formulation

100 g of fluid after-shower milk for the body contain:

| | | |
|---|---|---|
| • Water | g | 65,8 |
| • Hydrogenated castor oil 40 (OE) | g | 20,0 |
| • Glycol extract of echinacea purpurea | g | 5.0 |
| • Vegetable glycerin | g | 3.5 |
| • Sodium alginate | g | 1.5 |
| • Acrylic copolimer | g | 1.0 |
| • Perfume | g | 0.8 |
| • Mentol | g | 0.5 |
| • Adelmidrol | g | 0.5 |
| • Deo-usnate | g | 0.3 |
| • Carbomer | g | 0.3 |
| • Sodium hydroxide | g | 0.3 |
| • Triclosan | g | 0.3 |
| • Undecilenic acid | g | 0.1 |
| • Jaluronic acid | g | 0.05 |
| • Trans-traumatic acid | g | 0.005 |
| • Biotin | g | 0.001 |
| • Phytosphingosine | g | 0.001 |

The efficacy of the composition as an object of this invention has been demonstrated in numerous clinical tests and observations. Some patients have been treated which were affected by relapsing forms of PV and partly resistant to previous and repeated topical antimycotic therapies, using a topical preparation in cream gel according to this invention.

The study included 20 patients, 11 men and 9 women of an age comprised between 17 and 78 years, which presented hyperchromic or hypochromic pityriasic lesions, in a variable numbers, sometimes circumscribed to certain body regions, at other times diffused. In all cases, a direct mycological examination turned out to be positive for *Malassezia sp.* 9 cases were of diffused forms involving a skin surface of over 30%, while the remaining 11 cases were zone localized forms (Table 1).

**Table 1**

| **DIFFUSED FORMS IN PV** | | **LOCALIZED FORMS IN PV** | |
|---|---|---|---|
| **POINT** | **No.** | **POINT** | **No.** |
| Back, trunk | 3 | Back | 3 |
| Back, roots of upper limbs | 1 | Trunk | 3 |
| Back, trunk, forearm flexing surface | 1 | Face | 1 |
| Back, abdomen | 1 | submammary folds | 1 |
| Neck, nape, regions above collar bones | 1 | Forearm flexing surface | 1 |
| Neck, shoulders, roots of upper limbs, armpits | 1 | Left flank | 1 |
| Forearm flexing surface, submammary folds, abdomen | 1 | Region above pubis | 1 |

The topical preparation was applied in corrispondence to the lesions twice daily for four weeks. The patients were subjected to clinical and laboratory controls after two weeks and at the end of the treatment. The subjects that had attained a clinical and microbiological recovery at the end of the treatment were subjected to a follow-up for one month, so as to verify the eventual incidence of relapses.

The results of these clinical analyses were summarized in the Table 2 to follow. The cream gel determined a complete clinical and microbiological resolution in 13 patients or in 65% of the cases, among which 4 patients affected by a diffused form of PV and 9 by a localized form. In 5 patients with a diffused form of PV, i.e. in 25% of the cases, a partial resolution was obtained, meaning that a complete recovery was observed at a few zones of the lesions and a persistency of the clinical picture with a positive mycological examination at other zones. However, even in these cases the clinical aspect of the lesions appeared to be considerably alleviated with respect to the initial one. In the remaining 2 patients (10% of the cases), which were affected by a form of a diffused and localized hyperchromic character, respectively, no modification of the clinical and microbiological picture was observed. No collateral effects were observed. The follow-up in the 13 patients attaining a complete recovery has shown a maintenance of the recovered status in 92% of the subjects. A relapse was observed in only one patient at the end of the treatment.

**Table 2**

| **PZ** | **SEX** | **AGE** | **START OF TREATMENT** | **MYCOLOG. EXAMIN.** | **END OF TREATMENT** | | **FOLLOW-UP** | |
|---|---|---|---|---|---|---|---|---|
| | | | **(Clinical form of PV)** | **(Malassezia)** | **Obj.** | **Myc.** | **Obj.** | **Myc.** |
| | | | | | **Res.** | **Res.** | **Res.** | **Res.** |
| 1 | F | 17 | Diffused hyperchromic | + | Partial Resolution | + | / | / |
| 2 | F | 43 | Diffused hyperchromic | + | Partial Resolution | + | / | / |
| 3 | M | 18 | Diffused hyper-hypochromic | + | Partial Resolution | + | / | / |
| 4 | M | 17 | Diffused hyperchromic | + | Persistence | + | / | / |
| 5 | M | 33 | Diffused hypochromic | + | Complete Resolution | - | No relapse | - |
| 6 | M | 31 | Localized hyperchromic | + | Complete Resolution | - | No relapse | - |
| 7 | F | 66 | Diffused hyper-hypochromic | + | Complete resolution | - | No relapse | - |
| 8 | M | 39 | Localized hyperchromic | + | Complete Resolution | - | No relapse | - |
| 9 | M | 27 | Localized hyperchromic | + | Persistence | + | / | / |
| 10 | M | 48 | Localized hypochromic | + | Complete Resolution | - | Relapse | + |
| 11 | F | 19 | Localized hypochromic | + | Complete Resolution | - | No relapse | - |
| 12 | F | 33 | Localized hypochromic | + | Complete Resolution | - | No relapse | - |
| 13 | M | 78 | Diffused hyperchromic | + | Complete Resolution | - | No relapse | - |
| 14 | F | 28 | Localized Hypochromic | + | Complete Resolution | - | No relapse | - |
| 15 | F | 46 | Diffused hypochromic | + | Complete Resolution | - | No relapse | - |
| 16 | M | 67 | Diffused hyperchromic | + | Partial resolution | + | / | / |
| 17 | M | 38 | Localized hypochromic | + | Complete resolution | - | No relapse | - |
| 18 | F | 25 | Localized Hypochromic | + | Complete Resolution | - | No relapse | - |
| 19 | F | 75 | Diffused hyperchromic | + | Partial Resolution | + | / | / |
| 20 | M | 18 | Localized hypochromic | + | Complete Resolution | - | No relapse | - |

The effect of the local therapy by applying the preparation of the Example 4 was also evaluated on dog. For this purpose, 20 dogs of different races, body weight comprised between 6 and 15 kg, age comprised between 12 and 48 months and belonging to both sexes, which presented a well defined clinical picture of bilateral external otitis of an erythematous-ceruminous type accompanied in various degrees by edema and pain, were subjected to a local treatment. In particular, 10 dogs received the preparation of the Exemple 4, while 10 dogs received a traditional treatment. The latter treatment was constituted by an association of ketoconazol (10 mg/ml), gentamycine (5 mg/ml) and prednisolone (5 mg/ml). The treatment was extended to 15 days for both groups, with twice daily repeated applications in the auricle duct.

In the following, an observation comprising itching, erithema and exudates was evaluated as an overall clinical parameter (OCP), and a count of colonies as a microbiological parameter (MP). As refers to the evaluation of the clinical parameter, a point grade value was assigned depending on the response to the treatment (4 = excellent response, 3 = good response, 2 = fair response, 1 = insufficient response). The overall clinical parameter was noted at a time of 0, 5 days, 10 days, and 15 days. On the other hand, the microbiological parameter was noted at the time 0 and at the end of the treatment (15 days).

The data obtained are recorded in the following table (Table 3):

**Table 3**

| Animal | Parameter | Traditional treatment | | | | Preparation of the example 4 | | | |
|---|---|---|---|---|---|---|---|---|---|
| | | T0 | T5 | T10 | T15 | T0 | T5 | T10 | T15 |
| 1 | PCC | | 1 | 1 | 3 | | | | |
| | PM | 56 | | | 2 | | | | |
| 2 | PCC | | 2 | 3 | 4 | | | | |
| | PM | 115 | | | 5 | | | | |
| 3 | PCC | | | | | | 3 | 4 | 4 |
| | PM | | | | | 64 | | | 2 |
| 4 | PCC | | 2 | 3 | 3 | | | | |
| | PM | 36 | | | 4 | | | | |
| 5 | PCC | | | | | | 2 | 4 | 4 |
| | PM | | | | | 74 | | | 1 |
| 6 | PCC | | | | | | 3 | 3 | 3 |
| | PM | | | | | 70 | | | 3 |
| 7 | PCC | | | | | | 1 | 2 | 2 |
| | PM | | | | | 46 | | | 0 |
| 8 | PCC | | 3 | 3 | 4 | | | | |
| | PM | 55 | | | 2 | | | | |
| 9 | PCC | | | | | | 3 | 3 | 4 |
| | PM | | | | | 83 | | | 4 |
| 10 | PCC | | 1 | 1 | 2 | | | | |
| | PM | 112 | | | 22 | | | | |
| 11 | PCC | | 3 | 2 | 3 | | | | |
| | PM | 43 | | | 3 | | | | |
| 12 | PCC | | | | | | 2 | 4 | 4 |
| | PM | | | | | 83 | | | 2 |
| 13 | PCC | | | | | | 1 | 1 | 1 |
| | PM | | | | | 41 | | | 25 |
| 14 | PCC | | 3 | 3 | 3 | | | | |
| | PM | 36 | | | 15 | | | | |
| 15 | PCC | | | | | | 4 | 3 | 4 |
| | PM | | | | | 28 | | | 0 |
| 16 | PCC | | | | | | 2 | 3 | 3 |
| | PM | | | | | 56 | | | 2 |
| 17 | PCC | | 2 | 2 | 4 | | | | |
| | Pm | 89 | | | 6 | | | | |
| 18 | PCC | | | | | | 3 | 4 | 4 |
| | PM | | | | | 38 | | | 1 |
| 19 | PCC | | 3 | 3 | 3 | | | | |
| | PM | 63 | | | 12 | | | | |
| 20 | PCC | | 2 | 3 | 4 | | | | |
| | PM | 56 | | | 2 | | | | |

As can be seen from the experimental data listed above, the compostion according to this invention presents substantial advantages with respect to the preparations with a purely antimycotic activity. These preparations act directly on the pathogenous fungus by a fungicidal and/or fungistatic action, but without re-balancing the microenvironment of the infection. The dermo-epidermic activity which characterizes the acute infective state thus persists in the topic antimycotic treatment, which is the cause for the relapse of the mycoses after the treatment itself. The composition of the invention is on the other hand capable of contributing to the maintenance of the integrity of the skin tissue and to the normalization of the dermo-epidermic hyper-reactivity, thanks to the action of the agonist of the receptor of type CB2 of the cannabinoids. In this sense, the composition of this invention shapes up as the true solution of the previously outlined problem, that is the necessity of a new treament of the mycosis and of a prevention of the relapse of the acute inflammation.

A further object of this invention is therefore the use of the composition described for the preparation of a medicament for the treatment of the skin surface mycosis depending on *Malassezia*/*Pityrosporum.* In particular, the composition of the invention is utilized for the preparation of a medicament for the treatment of *Pityriasis versicolor* of human.

The *Malassezia pachydermatis,* one of the species of lipophile yeasts belonging to the *Malassezia* genus, is responsible for the itching dermatitis and for the otites in cats and dogs. The data reported in the biological example concerning the experimentation on the external otites of the dog show that it is possible to obtain, by using the preparation of the Example 4, results comparable with the much more toxic traditional association of antibiotics, antimycotics and cortisonics. This is both in terms of a clear improvement of the clinical parameters and of a drastic reduction of the microbic charge measured as a number of colonies, to the point of reaching the parameters considered physiological in a few days of treatment. A further aspect of this invention is therefore the use of the described composition for the preparation of a medicament for veterinary use for the treatment of the otitis and dermatitis associated with *Malassezia* in cats and dogs.

Moreover, the composition of this invention has a very important effect as regards the control of the excesses of sebum on which *Malassezia*/*Pityrosporum* encounters favourable conditions of growth. The fatty acid sequestering resin in fact deprives the pathogenous fungi of the nutritional ground, thereby blocking their development and differentiation. This results in a product with a medical activity which is not due to a primary pharmacological action directed against the pathogenous agent, but mediated by the sequestering of the nourishment for the pathogenous microorganism, with its consequent impossibility to grow and proliferate. This aspect allows inserting the composition of the invention among the products defined by the legislator as "medical devices".

Thanks to the action mechanism of the fatty acid sequestering resins which act, as mentioned, by depriving the pathogenous lipohile fungi of their nourishment, these agents may effectively be employed for the prevention and treatment of mycotic surface pathologies supported by strains of lipophile fungi even in association with traditional therapy. This sequestering agent will, if present in a traditional antimycotic composition, allow reducing the effective quantity of antimycotic, with the immediate result of knocking down the toxicity of the composition. In fact, the antimycotic preparations commonly known turn out to be toxic, especially if applied to a damaged and reddened skin, because of the high dosage of traditional antifungal substances contained in the same. In the antimycotic preparations it is therefore possible to exploit the association of a substance with a classic fungistatic/fungicidal action and of a resin as described in this invention.

A second further object of this invention is therefore the use of a free fatty acid sequestering resin in the preparation of an pharmaceutical antimycotic composition and of a formulation as outlined in claim 23.

It was additionally found that the composition of this invention may, because of its low toxicity, effectively be utilized even in the cosmetic sector. A further object of this invention is therefore a cosmetic formulation, capable of protecting or maintaining the outer skin surface in a good condition, comprising a composition as described in this invention in combination with other cosmetic additives, chosen depending on the type of formulation desired.

## Claims

1. A composition comprising a mixture of an agonist of the CB2-type receptor of cannabinoids and of a fatty acid sequestering resin, wherein said agonist of the CB2-type receptor of the cannabinoids is a compound of the general formula (I):
R₁-CO-NR₂R₃
wherein:
a) R₁ may be:
- an linear or branched alkylic, an alkylenic or arylic radical.
- a group of the formula:
-R₄-CO-NR₅R₆
wherein R₄ is a linear or branched alkylenic radical or an arylic biradical; R₅ and R₆ have the significance reported in the following for R₂ and R₃, respectively.
b) R₂ is chosen among hydrogen or an alkylic, alkenylic or arylic radical.
c) R₃ is a group of the formula.
-Y-OH
wherein Y is a linear or branched alkylenic radical, and said resin is an acrylic copolymer charged with phytosphingosine.

2. A composition according to claim 1, wherein said agonist of the CB2-type receptor of the cannabinoids and said resin are present in a weight ratio variable between 3:1 and 1:6, respectively.

3. A composition according to claim 2, wherein the weight ratio between the agonist of the CB2-type receptor of the cannabinoids and the resin is comprised between 1:1 and 1:2.

4. A composition according to any one of the claims from 1 to 3, wherein said agonist of the CB2-type receptor of the cannabinoids is a compound of the general formula (II):
HO-(CH₂)ₙ-NH-CO-(CH₂)ₘ-CO-NH-(CH₂)ₙ-OH
wherein n is a number which may vary between 2 and 10 and m is a number which may vary between 2 and 12.

5. A composition according to claim 4, wherein in said compound of a general formula (II) n is a number comprised between 2 and 4, and m is a number comprised between 5 and 9.

6. A composition according to the claims 4 and 5, wherein in said compound of a general formula (II) the value of n = 2 and the value of m = 7, and in which said compound is therefore adelmidrol.

7. A composition according to any claim 1 to 6, wherein said acrylic copolymer is POLYTRAP^{®} Q5-6603.

8. A composition according to any one of the claims from 1 to 7, comprising in addition one or more substances chosen among:
- a substance capable of sustaining and enhancing the keratinocytic metabolism, which is biotine;
- a substance with restructuring and bioadhesive action chosen among: jaluronic acid, traumatic acid or a mixture thereon;
- a substance with an antimycotic action chosen among: Echinacea purpurea, usnic acid, undecilenic acid, bromopol and triclosan or a mixture thereof.

9. A composition according to claim 8, wherein the substance capable of sustaining and enhancing the keratinocytic metabolism is present in a weight ratio between 1:50 and 1:250 with respect to the agonist of the CB2-type receptor of the cannabinoids.

10. A composition according to claim 8, wherein the substances with restructuring and bioadhesive action are present in a mixture of them in a weight ratio between 1:1,5 and 1:4 with respect to the agonist of the CB2-type receptor of the cannabinoids.

11. A composition according to claim 8, wherein the purple echinacea is, if present, in a weight ratio between 7:1 and 4:1 with respect to the agonist of the CB2-type receptor of the cannabinoids.

12. A composition according to claim 8, wherein
the usnic acid is, if present, in a weight ratio between 1:5 and 1:7 with respect to the agonist of the CB2-type receptor of the cannabinoids.

13. A composition according to claim 8, wherein
the undecilenic acid is, if present, in a weight ratio between 1:8 and 1:12 with respect to the agonist of the CB2-type receptor of the cannabinoids.

14. A composition according to claim 8, wherein
the bronopol is, if present, in a weight ratio between 1:30 and 1:50 with respect to the agonist of the CB2-type receptor of the cannabinoids.

15. A composition according to claim 8, wherein
the triclosan is, if present, in a weight ratio between 1:5 and 1:8 with respect to the agonist of the CB2-type receptor of the cannabinoids.

16. A pharmaceutical composition comprising a composition according to any one of the claims from 1 to 15, in combination with pharmaceutically acceptable excipients.

17. A pharmaceutical composition according to claim 16 for administration by a topic route, including the transdermic route.

18. The use of a pharmaceutical composition according to the claims 16 or 17 for the preparation of a medicament for human use for the treatment of skin surface mycoses.

19. The use of a pharmaceutical composition according to claim 18, wherein the surface mycosis is caused by yeast of the *Malassezia*/*Pityrosporum* genus.

20. The use of a pharmaceutical composition according to the claims 18 or 19, wherein the surface mycosis is *Pityriasis versicolor.*

21. The use of a pharmaceutical composition according to the claims 16 or 17, for the preparation of a medicament for veterinary use for the treatment of itching dermatitis and for the treatment of otitis in cats and dogs.

22. A cosmetic formulation comprising the composition according to any one of the claims from 1 to 15, in combination with other cosmetic additives.

23. Use of an acrylic copolymer, charged with phytosphingosine for the preparation of a medicament for human or veterinary use, having an antimycotic action.

## Patentansprüche

1. Eine Zusammensetzung, die ein Gemisch aus einem Agonisten des CB2-Typ-Rezeptors von Cannabinoiden und aus einem Fettsäure-Maskierungsmittel umfasst, wobei der Agonist des CB2-Typ-Rezeptors der Cannabinoide eine Verbindung der allgemeinen Formel (I) ist:
R₁-CO-NR₂R₃
wobei:
a) R₁ Folgendes sein kann:
- ein linearer oder verzweigter alkylischer, ein alkylenischer oder ein arylischer Radikal.
- eine Gruppe der Formel:
-R₄-CO-NR₅R₆
wobei R₄ ein linearer oder verzweigter alkylenischer Radikal oder ein arylischer Biradikal ist; R₅ und R₆ die Bedeutung aufweisen, die im Folgenden für R₂ beziehungsweise R₃ angegeben ist.
b) R₂ aus Wasserstoff oder einem alkylischen, alkenylischen oder arylischen Radikal ausgewählt ist.
c) R₃ eine Gruppe der Formel
-Y-OH
ist, wobei Y ein linearer oder verzweigter alkylenischer Radikal ist und das Harz ein acrylisches Copolymer ist, das mit Phytosphingosin besetzt ist.

2. Eine Zusammensetzung gemäß Anspruch 1, bei der der Agonist des CB2-Typ-Rezeptors der Cannabinoide und das Harz in einem Gewichtsverhältnis vorliegen, das zwischen 3:1 bzw. 1:6 variabel ist.

3. Eine Zusammensetzung gemäß Anspruch 2, bei der das Gewichtsverhältnis zwischen dem Agonist des CB2-Typ-Rezeptors der Cannabinoide und dem Harz zwischen 1:1 und 1:2 liegt.

4. Eine Zusammensetzung gemäß einem der Ansprüche 1 bis 3, bei der der Agonist des CB2-Typ-Rezeptors der Cannabinoide eine Verbindung der allgemeinen Formel (II) ist:
HO-(CH₂)ₙ-NH-CO-(CH₂)ₘ-CO-NH-(CH₂)ₙ-OH
wobei n eine Zahl ist, die zwischen 2 und 10 variieren kann, und m eine Zahl ist, die zwischen 2 und 12 variieren kann.

5. Eine Zusammensetzung gemäß Anspruch 4, bei der bei der Verbindung einer allgemeinen Formel (II) n eine Zahl ist, die zwischen 2 und 4 liegt, und m eine Zahl ist, die zwischen 5 und 9 liegt.

6. Eine Zusammensetzung gemäß den Ansprüchen 4 und 5, bei der bei der Verbindung einer allgemeinen Formel (II) der Wert von n = 2 und der Wert von m = 7 gelten und bei der die Verbindung somit Adelmidrol ist.

7. Eine Zusammensetzung gemäß einem der Ansprüche 1 bis 6, bei der das acrylische Copolymer POLYTRAP^{®} Q5-6603 ist.

8. Eine Zusammensetzung gemäß einem der Ansprüche 1 bis 7, die zusätzlich eine oder mehrere Substanzen umfasst, die aus den Folgenden ausgewählt ist beziehungsweise sind:
- einer Substanz, die in der Lage ist, den Keratinozyt-Stoffwechsel beizubehalten und zu verbessern, die Biotin ist;
- einer Substanz mit einer Umstrukturierungs- und bioadhäsiven Wirkung, die aus Folgenden ausgewählt ist: Jaluronsäure, Traumatinsäure oder einem Gemisch derselben;
- einer Substanz mit einer antimykotischen Wirkung, die aus Folgenden ausgewählt ist:
Echinacea purpurea, Usninsäure, Undecilensäure, Bromopol und Triclosan oder einem Gemisch derselben.

9. Eine Zusammensetzung gemäß Anspruch 8, bei der die Substanz, die in der Lage ist, den Keratinozyt-Stoffwechsel beizubehalten und zu verbessern, in, einem Gewichtsverhältnis zwischen 1:50 und 1:250 bezüglich des Agonisten des CB2-Typ-Rezeptors der Cannabinoide vorliegt.

10. Eine Zusammensetzung gemäß Anspruch 8, bei der die Substanzen mit einer Umstrukturierungs- und bioadhäsiven Wirkung in einem Gemisch derselben in einem Gewichtsverhältnis zwischen 1:1,5 und 1:4 bezüglich des Agonisten des CB2-Typ-Rezeptors der Cannabinoide vorliegen.

11. Eine Zusammensetzung gemäß Anspruch 8, bei der das purpurne Echinacea, falls es vorhanden ist, in einem Gewichtsverhältnis zwischen 7:1 und 4:1 bezüglich des Agonisten des CB2-Typ-Rezeptors der Cannabinoide vorliegt.

12. Eine Zusammensetzung gemäß Anspruch 8, bei der die Usninsäure, falls sie vorhanden ist, in einem Gewichtsverhältnis zwischen 1:5 und 1:7 bezüglich des Agonisten des CB2-Typ-Rezeptors der Cannabinoide vorliegt.

13. Eine Zusammensetzung gemäß Anspruch 8, bei der die Undecilensäure, falls sie vorhanden ist, in einem Gewichtsverhältnis zwischen 1:8 und 1:12 bezüglich des Agonisten des CB2-Typ-Rezeptors der Cannabinoide vorliegt.

14. Eine Zusammensetzung gemäß Anspruch 8, bei der das Bronopol, falls es vorhanden ist, in einem Gewichtsverhältnis zwischen 1:30 und 1:50 bezüglich des Agonisten des CB2-Typ-Rezeptors der Cannabinoide, vorliegt.

15. Eine Zusammensetzung gemäß Anspruch 8, bei der das Triclosan, falls es vorhanden ist, in einem Gewichtsverhältnis zwischen 1:5 und 1:8 bezüglich des Agonisten des CB2-Typ-Rezeptors der Cannabinoide vorliegt.

16. Eine pharmazeutische Zusammensetzung, die eine Zusammensetzung gemäß einem der Ansprüche 1 bis 15 in Kombination mit pharmazeutisch akzeptablen Bindemitteln umfasst.

17. Eine pharmazeutische Zusammensetzung gemäß Anspruch 16 zur Verabreichung auf einem die örtliche Lage betreffenden Weg, einschließlich des transdermalen Weges.

18. Die Verwendung einer pharmazeutischen Zusammensetzung gemäß den Ansprüchen 16 oder 17 zur Herstellung eines Medikaments zur Verwendung beim Menschen zur Behandlung von Mykosen der Hautoberfläche.

19. Die Verwendung einer pharmazeutischen Zusammensetzung gemäß Anspruch 18, bei der die Oberflächen-Mykose durch eine Hefe der Gattung *Malassezia*/*Pityrosporum* verursacht wird.

20. Die Verwendung einer pharmazeutischen Zusammensetzung gemäß den Ansprüchen 18 oder 19, bei der die Oberflächen-Mykose *Pityriasis versicolor* ist.

21. Die Verwendung einer pharmazeutischen Zusammensetzung gemäß den Ansprüchen 16 oder 17 zur Herstellung eines Medikaments zur Verwendung in der Tiermedizin zur Behandlung einer juckenden Dermatitis und zur Behandlung von Otitis bei Katzen und Hunden.

22. Eine kosmetische Formulierung, die die Zusammensetzung gemäß einem der Ansprüche 1 bis 15 in Kombination mit anderen kosmetischen Zusatzstoffen umfasst.

23. Verwendung eines acrylischen Copolymers, das mit Phytosphingosin besetzt ist, zur Herstellung eines Medikaments zur Verwendung beim Menschen oder in der Tiermedizin, das eine antimykotische Wirkung aufweist.

## Revendications

1. Composition comprenant un mélange d'un agoniste du récepteur des cannabinoïdes de type CB2 et d'une résine séquestrante d'acide gras, dans laquelle ledit agoniste du récepteur des cannabinoïdes de type CB2 est un composé de formule générale (I) :
R₁-CO-NR₂R₃
Dans laquelle :
a) R₁ peut être :
- un radical alkyle linéaire ou ramifié, alkylène ou aryle,
- un groupe de formule :
-R₄-CO-NR₅R₆
Dans lequel R₄ est un radical alkylène linéaire ou ramifié ou un biradical aryle ; R₅ et R₆ ont la signification mentionnée suivante pour R₂ et R₃, respectivement,
b) R₂ est choisi parmi l'hydrogène ou un radical alkyle, alcényle ou aryle,
c) R₃ est un groupe de formule
-Y-OH
Dans lequel Y est un radical alkylène linéaire ou ramifié, et ladite résine est un copolymère acrylique chargé avec la phytosphingosine.

2. Composition selon la revendication 1, dans laquelle ledit agoniste du récepteur des cannabinoïdes de type CB2 et ladite résine sont présents dans un rapport pondéral dans la gamme entre 3 : 1 et 1 : 6, respectivement.

3. Composition selon la revendication 2, dans laquelle le rapport pondéral de l'agoniste du récepteur de type CB2 des cannaboïdes à la résine est compris entre 1 : 1 et 1 : 2.

4. Composition selon l'une quelconque des revendications 1 à 3, dans laquelle ledit agoniste du récepteur de type CB2 des cannaboïdes est un composé de formule générale (II) :
HO-(CH₂)ₙ-NH-CO-(CH2)ₘ-CO-NH-(CH₂)ₙ-OH
Dans laquelle n est un nombre qui peut être dans la gamme entre 2 et 10 et m est un nombre qui peut être dans la gamme entre 2 et 12.

5. Composition selon la revendication 4, dans laquelle dans ledit composé de formule générale (II) n est un nombre compris entre 2 et 4, et m est un nombre compris entre 5 et 9.

6. Composition selon les revendications 4 et 5, dans laquelle dans ledit composé de formule générale (II) la valeur de n = 2 et la valeur de m = 7, et dans laquelle ledit composé est en conséquence l'adelmidrol.

7. Composition selon l'une quelconque des revendications 1 à 6, dans laquelle ledit copolymère acrylique est le POLYTRAP® Q5-6603.

8. Composition selon l'une quelconque des revendications 1 à 7, comprenant en outre une ou plusieurs des substances choisies parmi:
- une substance apte à contrôler et accroître le métabolisme kératinolytique, qui est la biotine ;
- une substance avec une activité restructurante et bioadhérente choisie parmi: l'acide jaluronique, l'acide traumatique ou un mélange de ceux-ci;
- une substance avec une activité antimycosique choisie parmi : Echinacea purpurea, l'acide usnique, l'acide undécilénique, le bromopol et le triclosan ou un mélange de ceux-ci.

9. Composition selon la revendication 8, dans laquelle la substance qui est capable de restructurer et d'accroître le métabolisme kératinolytique est présente dans un rapport pondéral entre 1 : 50 et 1 : 250 par rapport à l'agoniste du récepteur de type CB2 des cannaboïdes.

10. Composition selon la revendication 8, dans laquelle les substances avec activité restructurante et bioadhérente sont présentes dans le mélange dans un rapport pondéral entre 1 : 1,5 et 1 : 4 par rapport à l'agoniste du récepteur de type CB2 des cannaboïdes.

11. Composition selon la revendication 8, dans laquelle, lorsqu'elle est présente, l'echinea pourpre est dans un rapport pondéral entre 7 : 1 et 4 : 1 par rapport à l'agoniste du récepteur de type CB2 des cannaboïdes.

12. Composition selon la revendication 8, dans laquelle, lorsqu'il est présent, l'acide usnique est dans un rapport pondéral entre 1 : 5 et 1 : 7 par rapport à l'agoniste du récepteur de type CB2 des cannaboïdes.

13. Composition selon la revendication 8, dans laquelle lorsqu'il est présent, l'acide undécilénique, est dans un rapport pondéral entre 1 : 8 et 1 : 12 par rapport au récepteur de type CB2 des cannaboïdes.

14. Composition selon la revendication 8, dans laquelle, lorsqu'il est présent, le bronopol est dans un rapport pondéral entre 1 : 30 et 1 : 50 par rapport au récepteur de type CB2 des cannaboïdes.

15. Composition selon la revendication 8, dans laquelle, lorsqu'il est présent, le triclosan est dans un rapport pondéral entre 1 : 5 et 1 : 8 par rapport à l'agoniste du récepteur de type CB2 des cannaboïdes.

16. Composition pharmaceutique comprenant une composition selon l'une quelconque des revendications 1 à 15, en association avec des excipients acceptables d'un point de vue pharmaceutique.

17. Composition selon la revendication 16, pour une administration par une voie topique, incluant la voie transdermique.

18. Composition selon les revendications 16 ou 17, pour la préparation d'un médicament pour une utilisation humaine dans le traitement des mycoses de surface de la peau.

19. Utilisation d'une composition pharmaceutique selon la revendication 18, dans laquelle la mycose de surface est provoquée par une levure du genre *Malassezia* / *Pityrosporum.*

20. Utilisation d'une composition pharmaceutique selon les revendications 18 ou 19, dans laquelle la mycose de surface est *Pityriasis versicolor.*

21. Utilisation d'une composition pharmaceutique selon 16 ou 17, pour la préparation d'un médicament destiné à une utilisation vétérinaire dans le traitement de la dermatite et dans le traitement de l'otite chez les chats et les chiens.

22. Préparation cosmétique comprenant la composition selon l'une quelconque des revendications 1 à 15, en association avec d'autres additifs cosmétiques.

23. Utilisation d'un copolymère acrylique, chargé avec de la phytosphingosine pour la préparation d'un médicament pour une utilisation humaine ou vétérinaire, ayant une activité antimycosique.
